# EUROPEAN PATENT SPECIFICATION

(11) **EP 3 454 299 B1**
(45) Date of publication and mention of the grant of the patent: **16.04.2025**
(21) Application number: 18188294.5
(22) Date of filing: 09.08.2018
(51) Int. Cl.: A61B 5/00, A61B 5/05, A61B 6/03, A61B 6/50, A61B 8/08, A61B 6/00, A61B 34/10, G06T 7/30, G06T 7/70, A61B 8/00, A61B 34/20

(54) **METHOD AND APPARATUS FOR PERFORMING FACIAL REGISTRATION**
VERFAHREN UND VORRICHTUNG ZUR DURCHFÜHRUNG EINER GESICHTSREGISTRIERUNG
PROCÉDÉ ET APPAREIL DE RÉALISATION D'UN ENREGISTREMENT FACIAL

(30) Priority: 10.08.2017 US 201715673833; 10.08.2017 US 201715673824
(43) Date of publication of application: 13.03.2019
(73) Proprietor: Biosense Webster (Israel) Ltd., Yokneam 2066717 (IL)
(72) Inventor: GOVARI, Assaf, 2066717 Yokneam (IL); GLINER, Vadim, 2066717 Yokneam (IL)
(74) Representative: Carpmaels & Ransford LLP

(56) References cited:
- EP-A1- 3 305 202
- US-A- 5 676 673
- US-A1- 2002 035 321
- US-A1- 2014 193 053
- DIAKOV G ET AL: "Automatische Registration des Patienten mit A-Mode-Ultraschall für computerunterstützte Chirurgie ; Funktionsnachweis im Labor ; Automatic registration of patients with A-mode ultrasound for computer-assisted surgery ; Laboratory proof of concept", HNO ; DEUTSCHE GESELLSCHAFT FÜR HALS-NASEN-OHREN-HEILKUNDE, KOPFUND HALS-CHIRURGIE, SPRINGER, BERLIN, DE, vol. 58, no. 11, 30 September 2010 (2010-09-30), pages 1067 - 1073, XP019858654, ISSN: 1433-0458, DOI: 10.1007/S00106-010-2171-1

## Description

### BACKGROUND

US 2014/193053 A1 relates to a system and method for image registration that includes tracking a scanner probe in a position along a skin surface of a patient. Image planes corresponding to the position are acquired. A three-dimensional volume of a region of interest is reconstructed from the image planes. A search of an image volume is initialized to determine candidate images to register the image volume with the three-dimensional volume by employing pose information of the scanner probe during image plane acquisition, and physical constraints of a pose of the scanner probe. The image volume is registered with the three-dimensional volume.

The article "Automatische Registration des Patienten mit A-Mode-Ultraschall für computerunterstützte Chirurgie; Automatic registration of patients with A-mode ultrasound for computer-assisted surgery" by G. Diakov et al., HNO, SPRINGER, BERLIN, DE, vol. 58, no. 11, 30 September 2010, pages 1067-1073, demonstrates that A-mode ultrasound allows a 3D surface profile of the os occipitale to be created which can be registered on preoperative patient CT data; the transducer is mechanically positioned with sub-millimeter accuracy on the patients occiput; from the sound echos a 3D surface is generated and registered to the preoperative CT images with the iterative closest point (ICP) algorithm.

### SUMMARY

The invention is defined in the independent claims. Further embodiments of the invention are defined in the dependent claims.

### BRIEF DESCRIPTION OF THE DRAWINGS

A more detailed understanding may be had from the following description, given by way of example in conjunction with the accompanying drawings wherein:
Figure 1 is a diagram of an example system for performing a facial registration;
Figure 2 is a flow diagram of an example conventional method for performing a facial registration;
Figure 3 is an example facial image for use during the facial registration method of Figure 2;
Figure 4 is a diagram of an example registration probe for use in the system of Figure 1;
Figure 5 is an expanded schematic diagram 500 of the electronics of the example registration probe of Figure 4 in operation with a target on the patient's head and the workstation;
Figure 6 is a flow diagram of an example method for performing a facial registration; and
Figure 7 is an example facial image for use during the facial registration method performed in Figure 6.

### DETAILED DESCRIPTION

The present application is related to method and apparatus for performing a facial registration. In particular, the present application is directed to a method and apparatus for performing a facial registration for an ear/nose/throat (ENT) procedure, such as a nasal dilation.

In general, a person typically has eight or so sinus openings, (e.g., the frontal, anterior ethmoidal, maxillary, and middle ethmoidal, for each side), although the number of openings varies from person to person. Each of those sinus openings include areas that are very small in diameter. Accordingly, when an opening becomes clogged for one reason or another, no drainage may occur from within the sinuses. In this case, problems can occur. For example, an accumulation of mucus can cause various health issues such as infections.

In order to treat these problems, a medical procedure such as sinus dilation may be used. Sinus dilation is a technique for increasing the size of the sinus passageway to provide a more unrestricted flow of fluids to alleviate sinus congestion. Sinus dilation is performed by using a tool that is inserted into the sinus cavity that includes a balloon which can be inflated. The balloon is inserted into the middle of the small sinus opening and dilated. This dilation applies pressure on the sinus opening to widen it (e.g., by reshaping the tissue structure of the sinus cavity). The opening remains at this increased size once the balloon is taken out, thus providing a larger passageway for fluid flow.

To perform the nasal dilation procedure, or any ENT procedure that is similar, it is important that the physician performing the procedure knows the location of the tool within the nasal cavity with a great degree of accuracy. The physician views an image of the patient's nasal cavities on a display screen and navigates within the patient's actual cavities by manipulating the tool, while looking at an image of the location of the tool on the screen. Any inaccuracies regarding the location of the tool within a patient's nasal cavity may cause the physician to damage the nasal cavity and/or to perform the procedure in the wrong place within the cavity. Therefore, it is very important that the location of the tool on the screen coincides accurately to where the actual tool is within the actual nasal cavity of the patient.

In order to ensure that the physician knows, with accuracy, the location within the nasal cavity where he or she is performing the procedure, a registration procedure is performed. The registration procedure allows the physician to view the image that is on the display screen and register locations on a patient's face in that image. Although a system, apparatus and method are described in more detail below for performing a registration, briefly the image that is displayed is from a computerized tomography (CT) scan that is taken of the patient's head area prior to the ENT procedure. The registration is then performed by an operator, (e.g., physician or other technician), using a registration probe that is placed on various locations of a patient's face. Furthermore, the patient's head is situated in a magnetic field. The registration probe's location is registered on the CT scan that is displayed.

Conventional registration techniques include the operator touching the registration probe to different areas on the patient's face. However, because the tissue is soft on the facial areas, the contact of the registration probe deflects that tissue and potentially causes an inaccuracy in registering that location. Additionally, soft human tissue has a tendency to swell and reduce, (potentially up to 10%), based upon the amount of moisture in it, the humidity, or the like. Therefore, even if the registration probe is placed in substantially the same location on the patient's face as is registered in the CT scan, that facial location may not be in the same spot with respect to the nasal cavities as it was when the CT scan was taken due to swelling or reduction of the soft tissue areas.

Accordingly, described herein is a method, apparatus and system for performing a facial registration. The facial registration is performed on rigid tissue, (such as bone), that does not deflect and is not subject to absorbing moisture in the same way softer tissue is prone to do. The apparatus includes the use of a forward looking ultrasound device in concert with a magnetic registration device in a registration probe.

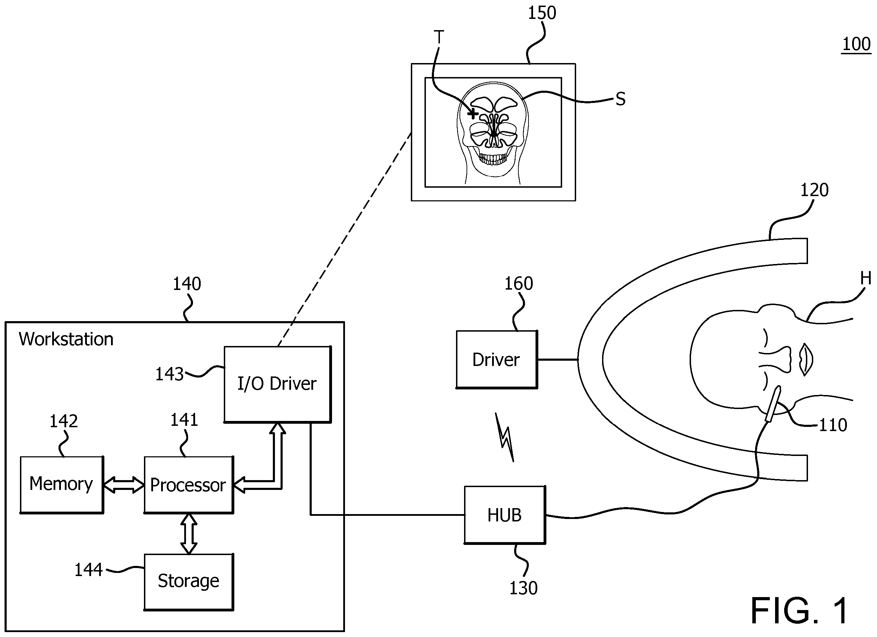

Figure 1 is a diagram of an example system 100 for performing a facial registration. The system includes a registration probe 110, a magnetic field emitter 120, a hub 130, a workstation 140, a display 150, and a magnetic driver 160. The registration probe 110 and the magnetic field emitter 120 may also be connected to the hub 130 via the magnetic driver 160, which is connected to the workstation 140, and receives the signals from both the registration probe 110 and the magnetic emitter 120 to transfer to the workstation 140. However, the registration probe 110 and the magnetic driver 160 may be in direct communication with the workstation 140, exclusive of a signal traversing through the hub 130.

The workstation 140 includes, for example, a processor 141, a memory 142, an input/output (I/O) driver 143, and storage 144, which allow the workstation 140 to receive input data and output data via the I/O driver 143, and store data in the storage 144 and/or memory 143 as needed for processing. The workstation 140 is also connected to the display 150. The driver 160 is connected to the magnetic emitter 120 to emit one or more magnetic fields and frequencies around a patient's head H. As can be seen on the display 150, an image S is displayed that has a target T on it. The target T indicates a current location of the registration probe 110 in space with respect to head H. An operator can then see the target on the image S and register areas of the patient's face on the image S for later use in an ENT procedure. It should be noted that the location of the magnetic field emitter 120 is shown for example purposes and the emitter 120 could be located in additional areas to provide a magnetic image. For example, a portion of the emitter 120 could be located beneath the patient's head H, and may include a plurality of magnetic field generators to increase the accuracy of the location. In the case where a plurality of field generators are utilized, the registration probe would be configured to register the plurality of magnetic fields.

To perform a registration, the registration probe 110 includes components to allow it to be accurately located with respect to the head H, and more particularly to the patient's face.

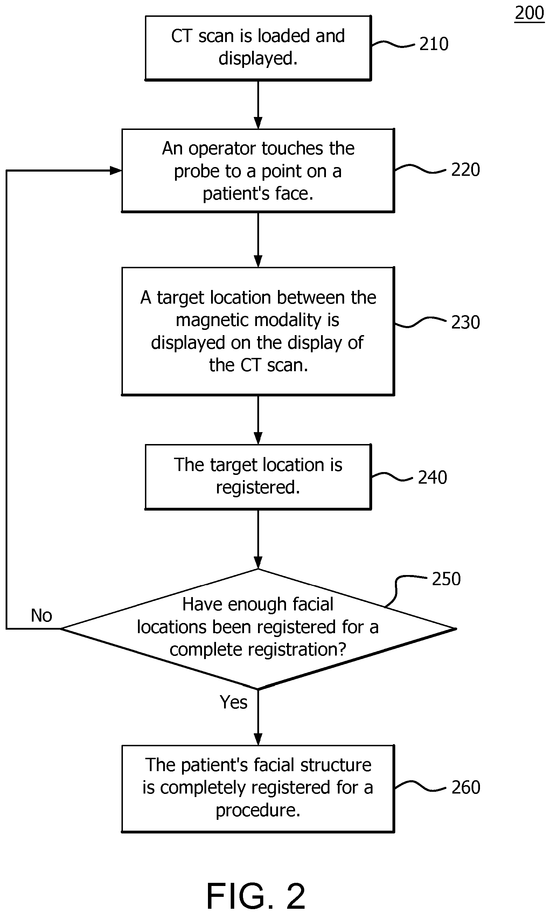

Figure 2 is a flow diagram of an example conventional method 200 for performing a facial registration. In step 210, a CT scan is loaded and displayed. For example, referring back to Figure 1, a CT scan file that has been captured previously and loaded onto a memory device or electronically sent is loaded into the workstation 140 and displayed on the display 150 as image S.

Once the image is displayed, an operator touches the registration probe to a reference point on the patient's face to register that target location on the image (step 220). For example, the operator touches registration probe 110 to an area of the patient's face depicted in the display 150 of Figure 1.

A target location between the magnetic modality is displayed on the CT scan (step 230). That is, the location that the coils 113 determine the registration probe 110 exists in three-dimensional space based on the magnetic field or fields received from the magnetic emitter 120 are displayed as a target location on the CT scan. This target location is then registered (step 240). This may be accomplished by the registration probe 110 transmitting its location information to the workstation 140 based on the detected magnetic fields by the coils 113, where the workstation 140 processes the location and determines where to overlay the location on the displayed image.

If enough facial locations have been registered for a complete registration (step 250), then the patient's facial structure is completely registered for the conventional procedure (step 260). If there are not enough locations registered in step 250, then the method returns to step 220, where the operator continues to touch other areas of the patient's face in order to cover a significant enough portion of the patient's face, (e.g., two-thirds), to completely register the patient's face for the ENT procedure.

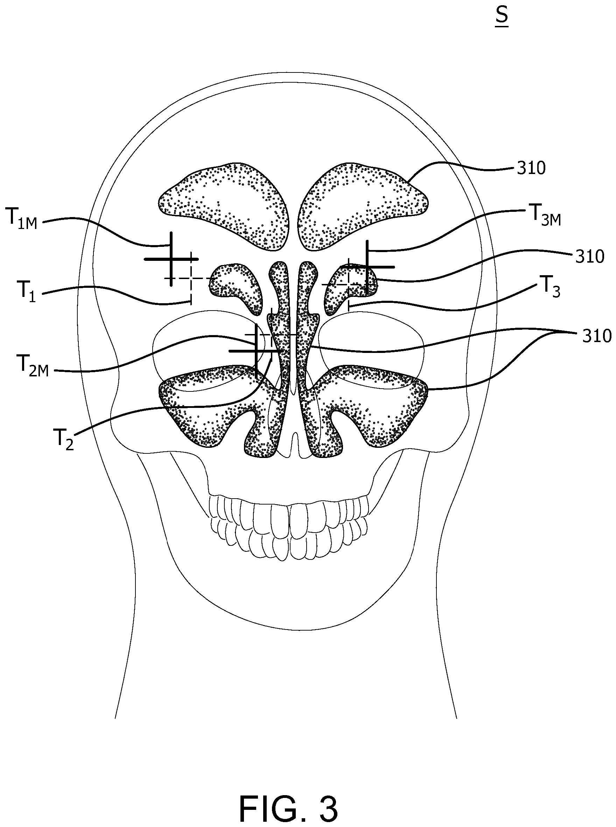

Figure 3 is an example facial image for use during the facial registration method of Figure 2. For purposes of example, the facial image in Figure 3 may be image S from Figure 1. As can be seen in Figure 3, targets T (designated T_{1M}, T_{2M}, and T_{3M} are shown as solid crosshairs. Referring back to step 220 of method 200, Target T_{1M} corresponds to a first point on the patient's face touched by the operator, target T_{2M} corresponds to a second point on the patient's face touched by the operator, and target T_{3M} corresponds to a third point on the patient's face touched by the operator. A number of nasal cavities 310 can also be seen in the image S as depicted in Figure 3. Also shown in Figure 3 are targets T₁, T₂, and T₃ (shown as dashed crosshairs). These targets represent the actual location of the probe 110 in three-dimensional space. As described previously, due to the error induced by utilizing a conventional registration method such as method 200 above, it can be seen that the registered targets T_{1M}, T_{2M}, and T_{3M} do not completely coincide with the actual target locations T₁, T₂, and T₃.

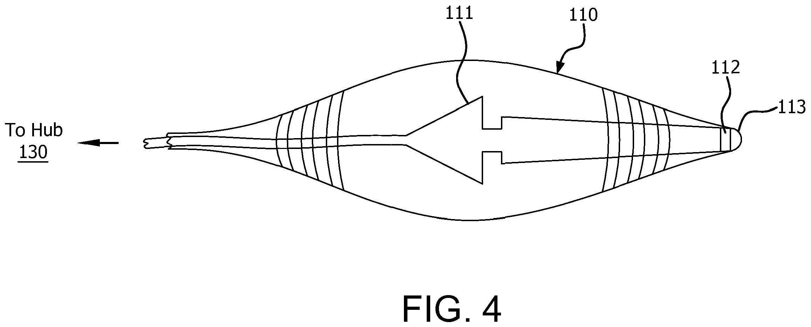

Figure 4 is a diagram of an example registration probe 110 for use in the system 100 of Figure 1. The registration probe 110 includes electronics 111 that operate the probe 110 and receive inputs from other components. The electronics 111 are connected to the hub 130 for eventual transmission to the workstation 140. The registration probe includes an ultrasonic emitter/receiver 112, which is a forward looking ultrasonic emitter/receiver, and coil 113. That is, the ultrasonic emitter/receiver 112 emits and receives an ultrasonic wave in an axial direction of the registration probe 110 toward the patient. The example registration probe 110 shown in Figure 4 may be formed of a transparent material (such as plastic), making visible the internal components. However, the probe can be formed of other non-transparent materials as well.

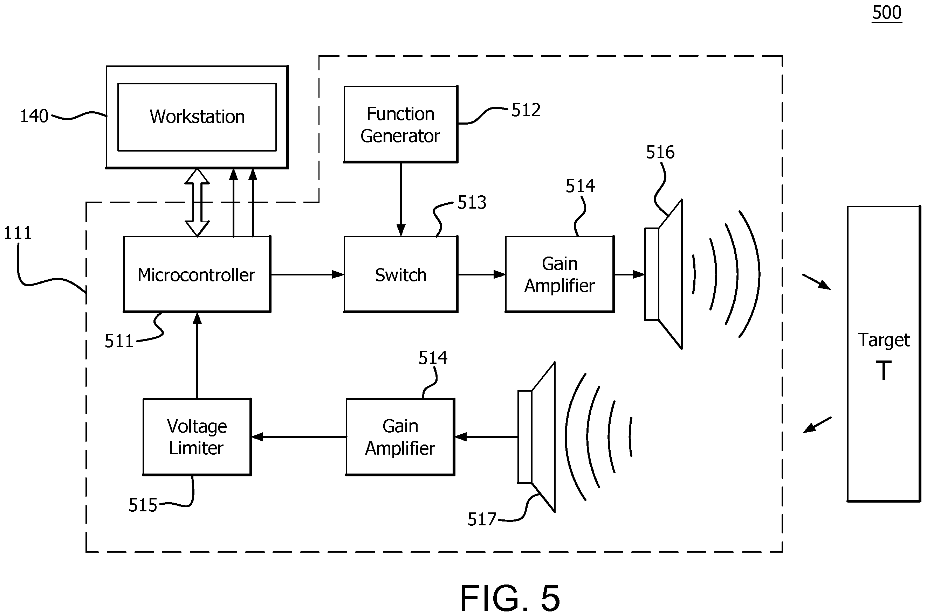

Figure 5 is an expanded schematic diagram 500 of the electronics 111 of the registration probe 110 in operation with a target T on the patient's head H and the workstation 140. As shown in Figure 5, the electronics 111 includes a microcontroller 511, function generator 512, switch 513, gain amplifiers 514, a voltage limiter 515, a transmitter 516 and a receiver 517. The microcontroller 511 is in communication with the workstation 140 and controls the transmitter 516 via the switch 513 to transmit ultrasonic waves. The microcontroller 511 receives the returned ultrasonic waves from the target T via the receiver 517 for transmission to the workstation 140. The function generator 512 generates an impulse for which a received echo is amplified by gain amplifiers 514. The voltage limiter 515 limits the voltage produced by the amplifier to avoid the sampling system becoming saturated.

The ultrasonic emitter/receiver 112 of the registration probe 110 emits ultrasonic waves which are reflected back, (e.g., from the bone surface of the patient's skull), and read to determine a location of the registration probe 110 with respect to the CT scan image S. The coil 113 receives the magnetic waves emitted by the magnetic emitter 120 to also locate the registration probe 110 in space with respect to the face of the head H. The coil 113 may be a series of windings (e.g., copper), that are arranged to receive the magnetic field emitted by the magnetic emitter in such a way to locate within the field where the registration probe 110 is.

As mentioned above, the accuracy of the registration is important because the ENT procedure performed will rely on an accurate registration to aid the physician performing the procedure in knowing where he or she is within the nasal cavity. The ultrasonic wave emitted and received by the ultrasonic emitter/receiver 112 of the registration probe 110 is correlated (described in more detail below) with the magnetic receiver, (i.e., the coil 113), in order to accurately locate the registration probe 110. This correlation then allows the physician performing the procedure to be able to locate the tool used for the procedure in the nasal cavity of the patient on the image S.

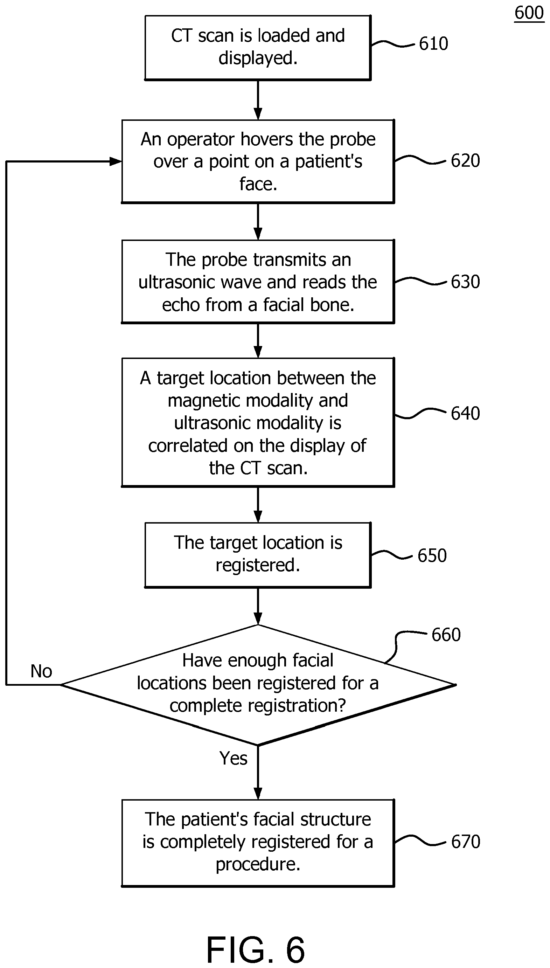

Figure 6 is a flow diagram of an example method 600 for performing a facial registration. In step 610, a CT scan is loaded and displayed. For example, referring back to Figure 1, a CT scan file that has been captured previously and loaded onto a memory device or electronically sent is loaded into the workstation 140 and displayed on the display 150 as image S.

Once the image is displayed, an operator hovers or touches the registration probe over a reference point on the patient's face to register that target location on the image (step 620). For example, the operator hovers registration probe 110 over an area of the patient's face depicted in Figure 1. The registration probe emits an ultrasonic wave which is echoed off of a rigid structure, such as a facial bone, and read by the registration probe (step 630). For example, the ultrasonic emitter/receiver 112 of the registration probe 110 emits the ultrasonic wave into the patient's face, which is then echoed off of a bone, or bony structure. The registration probe 110 then reads the echo/return of the wave via the ultrasonic emitter/receiver 112 to determine the location of the target T in the image S.

Once the ultrasonic wave is received, a target location between the magnetic modality and the ultrasonic modality is correlated on the CT scan (step 640). That is, the ultrasonic wave received by ultrasonic emitter/receiver 112 of the registration probe 110 is correlated to the location that the coils 113 determine the registration probe 110 exists in three-dimensional space based on the magnetic field received from the magnetic emitter 120. This correlation allows an accurate target location T to be determined and registered (step 650). This may be accomplished by the registration probe 110 transmitting its location information to the workstation 140, where the workstation 140 processes the location and determines where to overlay the location on the displayed image.

If enough facial locations have been registered for a complete registration (step 660), then the patient's facial structure is completely registered for the procedure (step 670). If there are not enough locations registered in step 660, then the method returns to step 620, where the operator continues to hover over other areas of the patient's face in order to cover a significant enough portion of the patient's face, (e.g., two-thirds), to completely register the patient's face for the ENT procedure. That is, for example, the operator may hover the registration probe over an area such as two-thirds of the patient's face. A physician monitoring the convergence of the probe 110 in real time on the image, (e.g., CT scan), may determine that a sufficient amount of the patient's face has been registered Alternatively, a mathematical algorithm, such as a minimum mean square error (MMSE) algorithm may be utilized to determine when enough of a patient's face has been registered.

Since the registration is bone to bone, (i.e., bone from the ultrasonic registration to bone in the CT scan), it is possible to know the distance from the tip of the registration probe 110 to the edge of the bone. Accordingly, the bone in the CT scan can be correlated to the bone detected by the ultrasonic wave. The soft tissue thickness can also be estimated in this manner. For example, if hovering on the bridge of a nose, the soft tissue appears rigid because the distance between the bony structure and facial surface is very small. This additional information can be utilized by a physician to determine whether or not enough of the face has been registered to the CT scan or mathematically by including the location in the MMSE algorithm described above in calculating the error.

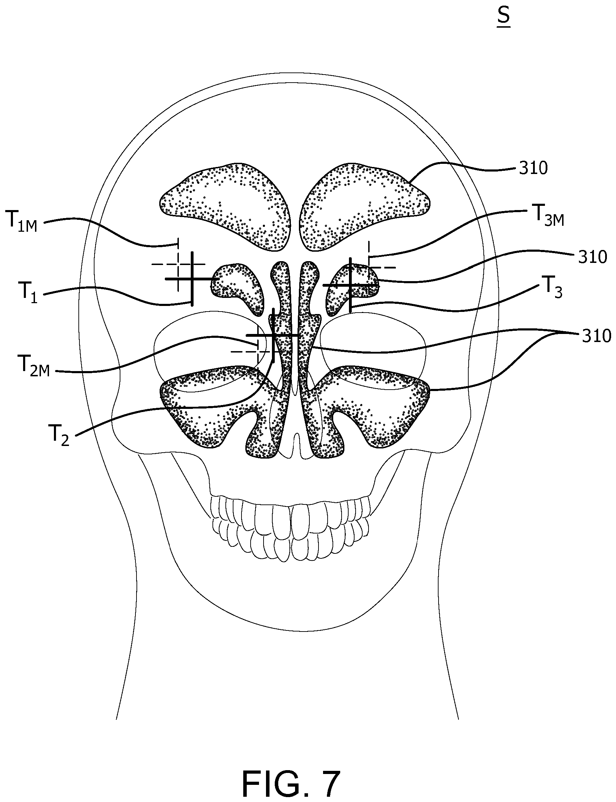

Figure 7 is an example facial image for use during the facial registration method 600 performed in Figure 6. For purposes of example, the facial image in Figure 7 may be image S from Figure 1. Additionally, the facial image in Figure 7 is substantially similar to that in Figure 3. As can be seen in Figure 7, targets T (designated T₁, T₂, and T₃) are shown as solid crosshairs. Referring back to step 640 of method 600, Target T₁ corresponds to a first correlated point on the patient's face registered by the operator, target T₂ corresponds to a second correlated point on the patient's face registered by the operator, and target T₃ corresponds to a third correlated point on the patient's face registered by the operator. As in Figure 3, a number of nasal cavities 310 can also be seen in the image S as depicted in Figure 7. Also shown in Figure 7 for example purposes are targets T_{1M}, T_{2M}, and T_{3M} (shown as dashed crosshairs). These targets represent the targets that would be registered in a conventional magnetic modality registration method, such as method 200 described above. Targets T₁, T₂, and T₃ represent the actual location of the probe 110 in three-dimensional space. It can therefore be seen then that an ENT procedure performed utilizing the correlated target locations acquired in method 600 would be performed in a correct area with relation to the nasal cavities 310 as compared to performing the procedure utilizing the target areas acquired in the conventional method 200.

Accordingly, above is described a forward looking ultrasound system near the distal end of a registration probe that generates some impact on human rigid tissue and waits for an echo. By calculating the amount of time that passes the registration system can determine where, (i.e., how deep), the tissue is. Since bony structures reflect virtually all of the ultrasound energy without absorbing any of it, it is easy to identify using ultrasound. By hovering the registration probe and utilizing the ultrasound registration along with the magnetic registration technique, a more accurate registration can be achieved.

It should be noted that the method, apparatus and system described above can include additional modifications. For example, the registration probe, (e.g., registration probe 110), can be a high-frequency probe that hovers on the surface of the skin with a matching impedance such as a gel, and leaves a gel trace. Additionally, components in communication with one another can be in wired or wireless communication. That is, transceivers and antennas may be included in the devices, (e.g., registration probe 110 and other components of system 100), that can transmit and receive data wirelessly to one another.

The methods provided can be implemented in a general purpose computer, a processor, or a processor core. Suitable processors include, by way of example, a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine. Such processors can be manufactured by configuring a manufacturing process using the results of processed hardware description language (HDL) instructions and other intermediary data including netlists (such instructions capable of being stored on a computer readable media). The results of such processing can be maskworks that are then used in a semiconductor manufacturing process to manufacture a processor which implements features of the disclosure.

The methods or flow charts provided herein can be implemented in a computer program, software, or firmware incorporated in a non-transitory computer-readable storage medium for execution by a general purpose computer or a processor. Examples of non-transitory computer-readable storage mediums include a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs).

## Claims

1. A method for performing facial registration on rigid tissue for an ear/nose/throat procedure, comprising:
loading (210, 610) a reference image (S) into a workstation; wherein a registration probe is in communication with the workstation; and
displaying the reference image on a display in communication with the workstation;
hovering (220, 620) the registration probe (110) over a plurality of target locations (T) on a face of a patient;
controlling, using a microcontroller, a transmitter to emit (630) an ultrasonic wave from the registration probe at each of the target locations;
receiving, via the registration probe, a return of the ultrasonic wave from each of the target locations;
receiving, via the registration probe, a magnetic signal from a magnetic emitter located proximate to the face of the patient to identify a location in space of the registration probe relative to the magnetic emitter; and
correlating (640) each of the target locations of the received ultrasonic return to a location identified in space relative to the magnetic emitter;
wherein correlating each of the target locations of the received ultrasonic return to a location identified in space relative to the magnetic emitter includes correlating each of the target locations on the reference image displayed; and
wherein the reference image (S) is a computerized tomography, CT, scan of the face of the patient, and wherein the reference image (S) includes an image of nasal cavities (310) of the patient and bone structure of the patient; and
wherein the ultrasonic return is a wave reflected from a location on the bone structure of the patient and is received to determine a location of the registration probe with respect to the reference image, which location is compared to the location in space of the registration probe (110) relative to the magnetic emitter to acquire a correlated target location.

2. An apparatus for performing facial registration on rigid tissue for an ear/nose/throat procedure, comprising a registration probe (110) and a workstation (140), the registration probe comprising:
an ultrasonic wave emitter (112, 516) configured to emit an ultrasonic wave at each of a target location (T) of a plurality of target locations on a face of a patient;
a microcontroller (511) configured to control the ultrasonic wave emitter (112, 516) to emit the ultrasonic wave;
an ultrasonic wave receiver (112, 517) configured to receive an ultrasonic return from each of the plurality of target locations; and
a magnetic wave receiver (113) configured to receive a magnetic signal from a magnetic emitter (120) located proximate to the face of the patient to identify a location in space of the registration probe relative to the magnetic emitter; wherein
the microcontroller is configured to transmit the ultrasonic return and the received magnetic signal to the workstation, wherein the workstation comprises a processor (141) configured to correlate the ultrasonic return and the received magnetic wave for each of the plurality of target locations to create a plurality of correlated target locations, wherein the microcontroller (511) is configured to transmit the ultrasonic return and the received magnetic signal for display on a reference image (S) of the face of the patient; and
wherein the reference image (S) is a computerized tomography, CT, scan of the face of the patient, and wherein the reference image (S) includes an image of nasal cavities (310) of the patient and bone structure of the patient; and
wherein the ultrasonic return is a wave reflected from a location on the bone structure of the patient, and wherein the workstation is configured to receive the ultrasonic return, and wherein the processor (141) is configured to determine a location of the registration probe with respect to the reference image, and to compare the location of the registration probe with respect to the reference image to the location in space of the registration probe (110) relative to the magnetic emitter to acquire a correlated target location.

3. The method of claim 1, further comprising registering the correlated target location on the reference image (S).

4. The method of claim 3 wherein the correlated target location is displayed on the reference image.

5. The registration probe of claim 2 wherein the correlated target location is correlated to a location on the bone structure on the reference image.

6. The registration probe of claim 2, further comprising a wireless transceiver for transmitting data wirelessly to a workstation.

7. The registration probe of claim 2 wherein the magnetic wave receiver (113) is a coil disposed in the registration probe for receiving the magnetic wave.

## Patentansprüche

1. Verfahren zum Durchführen einer Gesichtsregistrierung auf starrem Gewebe für einen Hals-Nasen-Ohren-Eingriff, umfassend:
Laden (210, 610) eines Referenzbilds (S) in eine Arbeitsstation; wobei eine Registrierungssonde mit der Arbeitsstation in Verbindung steht; und
Anzeigen des Referenzbilds auf einer Anzeige, die mit der Arbeitsstation in Verbindung steht;
Schweben (220, 620) der Registrierungssonde (110) über einer Vielzahl von Zielorten (T) auf einem Gesicht eines Patienten;
Steuern, unter Verwendung einer Mikrosteuerung, eines Senders, um an jedem der Zielorte eine Ultraschallwelle von der Registrierungssonde zu emittieren (630);
Empfangen, über die Registrierungssonde einer Rückmeldung der Ultraschallwelle von jedem der Zielorte;
Empfangen, über die Registrierungssonde, eines magnetischen Signals von einem Magnetemitter, der sich in der Nähe des Gesichts des Patienten befindet, um einen Ort in einem Raum der Registrierungssonde relativ zu dem Magnetemitter zu identifizieren; und
Korrelieren (640) jedes der Zielorte der empfangenen Ultraschallrückmeldung mit einem Ort, der in dem Raum relativ zu dem Magnetemitter identifiziert wird;
wobei das Korrelieren jedes der Zielorte der empfangenen Ultraschallrückmeldung mit einem Ort, der in dem Raum relativ zu dem Magnetemitter identifiziert wird, das Korrelieren jedes der Zielorte auf dem angezeigten Referenzbild umfasst; und
wobei das Referenzbild (S) ein Computertomographie-Scan, CT-Scan, des Gesichts des Patienten ist und wobei das Referenzbild (S) ein Bild der Nasenhöhlen (310) des Patienten und der Knochenstruktur des Patienten einschließt; und
wobei die Ultraschallrückmeldung eine von einem Ort auf der Knochenstruktur des Patienten reflektierte Welle ist und empfangen wird, um einen Ort der Registrierungssonde in Bezug auf das Referenzbild zu bestimmen, wobei der Ort mit dem Ort in dem Raum der Registrierungssonde (110) relativ zu dem Magnetemitter verglichen wird, um einen korrelierten Zielort zu erhalten.

2. Vorrichtung zum Durchführen einer Gesichtsregistrierung auf starrem Gewebe für einen Hals-Nasen-Ohren-Eingriff, umfassend eine Registrierungssonde (110) und eine Arbeitsstation (140), die Registrierungssonde umfassend:
einen Ultraschallwellenemitter (112, 516), der konfiguriert ist, um an jedem Zielort (T) einer Vielzahl von Zielorten auf einem Gesicht eines Patienten eine Ultraschallwelle zu emittieren;
eine Mikrosteuerung (511), die konfiguriert ist, um den Ultraschallwellenemitter (112, 516) zu steuern, um die Ultraschallwelle zu emittieren;
einen Ultraschallwellenempfänger (112, 517), der konfiguriert ist, um eine Ultraschallrückmeldung von jedem der Vielzahl von Zielorten zu empfangen; und
einen Magnetwellenempfänger (113), der konfiguriert ist, um ein Magnetsignal von einem Magnetemitter (120) zu empfangen, der sich in der Nähe des Gesichts des Patienten befindet, um einen Ort in einem Raum der Registrierungssonde relativ zu dem Magnetemitter zu identifizieren; wobei
die Mikrosteuerung konfiguriert ist, um die Ultraschallrückmeldung und das empfangene Magnetsignal an die Arbeitsstation zu senden, wobei die Arbeitsstation einen Prozessor (141) umfasst, der konfiguriert ist, um die Ultraschallrückmeldung und die empfangene Magnetwelle für jeden der Vielzahl von Zielorten zu korrelieren, um eine Vielzahl von korrelierten Zielorten zu erzeugen, wobei die Mikrosteuerung (511) konfiguriert ist, um die Ultraschallrückmeldung und das empfangene Magnetsignal für die Anzeige auf einem Referenzbild (S) des Gesichts des Patienten zu senden; und
wobei das Referenzbild (S) ein Computertomographie-Scan, CT-Scan, des Gesichts des Patienten ist und wobei das Referenzbild (S) ein Bild der Nasenhöhlen (310) des Patienten und der Knochenstruktur des Patienten einschließt; und
wobei die Ultraschallrückmeldung eine Welle ist, die von einem Ort auf der Knochenstruktur des Patienten reflektiert wird, und wobei die Arbeitsstation konfiguriert ist, um die Ultraschallrückmeldung zu empfangen, und wobei der Prozessor (141) konfiguriert ist, um einen Ort der Registrierungssonde in Bezug auf das Referenzbild zu bestimmen und den Ort der Registrierungssonde in Bezug auf das Referenzbild mit dem Ort in dem Raum der Registrierungssonde (110) relativ zu dem Magnetemitter zu vergleichen, um einen korrelierten Zielort zu erhalten.

3. Verfahren nach Anspruch 1, ferner umfassend das Registrieren des korrelierten Zielorts auf dem Referenzbild (S).

4. Verfahren nach Anspruch 3, wobei der korrelierte Zielort auf dem Referenzbild angezeigt wird.

5. Registrierungssonde nach Anspruch 2, wobei der korrelierte Zielort mit einem Ort auf der Knochenstruktur auf dem Referenzbild korreliert ist.

6. Registrierungssonde nach Anspruch 2, ferner umfassend einen drahtlosen Sender zum drahtlosen Senden von Daten an eine Arbeitsstation.

7. Registrierungssonde nach Anspruch 2, wobei der Magnetwellenempfänger (113) eine Spule ist, die in der Registrierungssonde zum Empfangen der Magnetwelle angeordnet ist.

## Revendications

1. Procédé permettant d'effectuer un enregistrement de visage sur un tissu rigide pour une intervention sur l'oreille/le nez/la gorge, comprenant :
le chargement (210, 610) d'une image de référence (S) dans un poste de travail ; dans lequel une sonde d'enregistrement est en communication avec le poste de travail ; et
l'affichage de l'image de référence sur un dispositif d'affichage en communication avec le poste de travail ;
le faire de faire planer (220, 620) la sonde d'enregistrement (110) au-dessus d'une pluralité d'emplacements cibles (T) sur un visage d'un patient ;
la commande, à l'aide d'un microcontrôleur, d'un émetteur afin d'émettre (630) une onde ultrasonique à partir de la sonde d'enregistrement au niveau de chacun des emplacements cibles ;
la réception, par l'intermédiaire de la sonde d'enregistrement, d'un retour de l'onde ultrasonique provenant de chacun des emplacements cibles ;
la réception, par l'intermédiaire de la sonde d'enregistrement, d'un signal magnétique provenant d'un émetteur magnétique situé à proximité du visage du patient, afin d'identifier un emplacement dans l'espace de la sonde d'enregistrement par rapport à l'émetteur magnétique ; et
la corrélation (640) de chacun des emplacements cibles du retour ultrasonique reçu avec un emplacement identifié dans l'espace par rapport à l'émetteur magnétique ;
dans lequel la corrélation de chacun des emplacements cibles du retour ultrasonique reçu avec un emplacement identifié dans l'espace par rapport à l'émetteur magnétique comporte la corrélation de chacun des emplacements cibles sur l'image de référence affichée ; et
dans lequel l'image de référence (S) est un balayage de tomographie par ordinateur, CT, du visage du patient, et dans lequel l'image de référence (S) comporte une image de cavités nasales (310) du patient et de la structure osseuse du patient ; et
dans lequel le retour ultrasonique est une onde réfléchie à partir d'un emplacement sur la structure osseuse du patient et est reçu pour déterminer un emplacement de la sonde d'enregistrement par rapport à l'image de référence, lequel emplacement est comparé à l'emplacement dans l'espace de la sonde d'enregistrement (110) par rapport à l'émetteur magnétique afin d'acquérir un emplacement cible corrélé.

2. Appareil permettant d'effectuer un enregistrement de visage sur un tissu rigide pour une intervention sur l'oreille/le nez/la gorge, comprenant une sonde d'enregistrement (110) et un poste de travail (140), la sonde d'enregistrement comprenant :
un émetteur d'onde ultrasonique (112, 516) configuré pour émettre une onde ultrasonique au niveau de chaque emplacement cible (T) d'une pluralité d'emplacements cibles sur un visage d'un patient ;
un microcontrôleur (511) configuré pour commander l'émetteur d'onde ultrasonique (112, 516) afin qu'il émette l'onde ultrasonique ;
un récepteur d'onde ultrasonique (112, 517) configuré pour recevoir un retour ultrasonique à partir de chacun de la pluralité d'emplacements cibles ; et
un récepteur d'onde magnétique (113) configuré pour recevoir un signal magnétique à partir d'un émetteur magnétique (120) situé à proximité du visage du patient afin d'identifier un emplacement dans l'espace de la sonde d'enregistrement par rapport à l'émetteur magnétique ; dans lequel
le microcontrôleur est configuré pour transmettre le retour ultrasonique et le signal magnétique reçu au poste de travail, dans lequel le poste de travail comprend un processeur (141) configuré pour corréler le retour ultrasonique et l'onde magnétique reçue pour chacun de la pluralité d'emplacements cibles afin de créer une pluralité d'emplacements cibles corrélés, dans lequel le microcontrôleur (511) est configuré pour transmettre le retour ultrasonique et le signal magnétique reçu pour un affichage sur une image de référence (S) du visage du patient ; et
dans lequel l'image de référence (S) est un balayage de tomographie par ordinateur, CT, du visage du patient, et dans lequel l'image de référence (S) comporte une image de cavités nasales (310) du patient et de la structure osseuse du patient ; et
dans lequel le retour ultrasonique est une onde réfléchie à partir d'un emplacement sur la structure osseuse du patient, et dans lequel le poste de travail est configuré pour recevoir le retour ultrasonique, et dans lequel le processeur (141) est configuré pour déterminer un emplacement de la sonde d'enregistrement par rapport à l'image de référence, et pour comparer l'emplacement de la sonde d'enregistrement par rapport à l'image de référence à l'emplacement dans l'espace de la sonde d'enregistrement (110) par rapport à l'émetteur magnétique afin d'acquérir un emplacement cible corrélé.

3. Procédé selon la revendication 1, comprenant en outre l'enregistrement de l'emplacement cible corrélé sur l'image de référence (S).

4. Procédé selon la revendication 3, dans lequel l'emplacement cible corrélé est affiché sur l'image de référence.

5. Sonde d'enregistrement selon la revendication 2, dans laquelle l'emplacement cible corrélé est corrélé à un emplacement sur la structure osseuse sur l'image de référence.

6. Sonde d'enregistrement selon la revendication 2, comprenant en outre un émetteur-récepteur sans fil permettant de transmettre des données sans fil à un poste de travail.

7. Sonde d'enregistrement selon la revendication 2, dans laquelle le récepteur d'onde magnétique (113) est une bobine disposée dans la sonde d'enregistrement permettant de recevoir l'onde magnétique.
